# EUROPEAN PATENT APPLICATION

(11) **EP 4 721 561 A1**
(43) Date of publication of application: **08.04.2026**
(21) Application number: 25206050.4
(22) Date of filing: 01.10.2025
(51) Int. Cl.: A01M 1/20

(54) **MOSQUITO REPELLENT COMPONENT, MOSQUITO REPELLENT USAGE KIT AND MOSQUITO REPELLENT**

(30) Priority: 02.10.2024 FI 20246186
(71) Applicant: Risja Oy, 91150 Yli-Olhava (FI)
(72) Inventor: RUUSKA, Jarmo, 91150 YLI-OLHAVA (FI)
(74) Representative: Berggren Oy

(57) **Abstract**

A component (36) for a mosquito repellent device comprises a heat resistant absorbent pad (10) for absorption and temporary storage of a mosquito repellent, and for diffusing it into the surrounding air. The component is re-usable, and it comprises a supporting structure protecting the pad from mechanical stress.

## Description

### Field of the invention

The invention relates to a mosquito repellent component comprising a heat resistant absorbent pad for absorption and temporary storage of a mosquito repellent, and for diffusing it into the surrounding air. The invention also comprises a mosquito repellent usage kit and a mosquito repellent device.

### Prior art

For repelling mosquitoes, various liquid repellents are available, which can be applied in liquid form onto the skin or clothing, or spread as a spray into the surrounding air. On clothing and on the skin, a repellent may feel uncomfortable, and it often leaves a pungent odour in clothing. Sprayed into the air, the repellent remains in the air for a short time only, after which its repellent effect ceases. Devices developed for repelling mosquitoes are also known, in which the repellent is absorbed into a felt-like pad, from which it is released as a fume into the surrounding air when the pad is heated. In normal use, the repellent is totally evaporated from the pad within a few hours, after which the pad must be replaced with a new one. Non-reusable repellent pads are expensive, making the costs for using the device relatively high. Moreover, a lot of waste is generated from used pads.

Document US 4,849,729 discloses a mosquito repellent assembly comprising a felt-like carrier element containing activated carbon fibers and volatile mosquito repellent. In the document, the carrier element is not heated to enhance the evaporation of the insect repellent.

Document US H1731 discloses a non-reusable mosquito repellent device which contains liquid mosquito repellent absorbed in a fibrous substance. The fibrous substance is placed in a housing having a porous surface layer, through which the repellent can evaporate from the casing.

Document EP1029451 A1 discloses a component with a pad placed in a recess and impregnated with liquid mosquito repellent.

Document US 2010247372 A1 discloses a non-reusable chemical delivery device comprising a housing, a heat source enclosed in the housing, and a chemical storage connected to the heat source and containing a volatile chemical, such as a mosquito repellent, absorbed in it. The chemical storage cannot be replaced or re-used.

It is an aim of the invention to present a mosquito repellent component, a mosquito repellent usage kit, and mosquito repellent, for reducing problems involved in the prior art. The aims of the invention are achieved with a component, a usage kit, and a mosquito repellent device, which are characterized by what is presented in the independent claims. Some advantageous embodiments of the invention are presented in the dependent claims.

### Brief summary of the invention

The invention relates to a mosquito repellent component comprising a heat resistant absorbent pad for absorption and temporary storage of a mosquito repellent, and for diffusing it into the surrounding air, as well as a supporting structure protecting said pad from mechanical stress. Said component is re-usable, and said pad comprises a first surface and a second surface, and said supporting structure has a first wall placed against the first surface of the pad, and a second wall placed against the second surface of the pad, the first and second walls being permeable to liquid. Thanks to the supporting structure, the component can be treated repeatedly without its pad for storing mosquito repellent being subjected to mechanical wear and stress. Thus, the supporting structure enables re-use of the component, that is, removal of the used composed from the repellent device, absorption of repellent into the pad, and returning the component into the repellent device. Permeability of the walls to liquid makes it possible to dispense liquid mosquito repellent into the pad through the wall. Correspondingly, vaporized repellent can exit the pad through the wall.

In an embodiment of the component according to the invention, said pad is a flexible, felt-like piece.

In an embodiment of the component according to the invention, the ignition or melting point of said pad is higher than 300°C, preferably higher than 500°C. The high ignition and melting points make it possible to heat the component to a temperature at which the mosquito repellent is vaporized and released as a fume into the surrounding air.

In an embodiment of the component according to the invention, said supporting structure and said pad are made of different materials.

In an embodiment of the component according to the invention, the ignition or melting point of said supporting structure is at least as high as the ignition or melting point of said pad. With the higher ignition or melting point of the supporting structure, it is secured that the supporting structure remains functional as long as the pad is functional.

In an embodiment of the component according to the invention, the first and/or the second wall comprises a mesh having a planar shape, a plurality of thin rigid threads in substantially the same plane, and/or a perforated sheet.

In an embodiment of the component according to the invention, said supporting structure encloses said pad substantially entirely.

In an embodiment of the component according to the invention, said first and second walls are connected to each other by at least one fastening means penetrating said pad.

In an embodiment of the component according to the invention, the rigidity and the bending strength of said supporting structure are higher than the rigidity and the bending strength of said pad.

In an embodiment of the component according to the invention, said supporting structure is at least partly made of metal, silicone, plastic resistant to a high temperature, or Teflon.

A mosquito repellent usage kit according to the invention comprises a mosquito repellent component according to any of the preceding claims, and a liquid package containing a liquid mosquito repellent.

In an embodiment of the usage kit according to the invention, the pad of the component has a capacity to absorb mosquito repellent, and said liquid package has a liquid volume, which liquid volume is multiple, preferably at least ten-fold, most preferably at least 50-fold, with respect to said absorption capacity. The same component can thus be re-used several times in the repellent device, by always impregnating the pad with another dose of liquid repellent after the preceding dose has diffused from the pad by evaporation.

The mosquito repellent device according to the invention comprises a space for housing a component comprising volatile mosquito repellent and a heating device for heating the component. Said component is a component according to the invention as described above.

The invention has the advantage of reducing usage costs of mosquito repellent devices, because the same component can be re-used several times by adding repellent from time to time.

Yet another advantage of the invention is that it is environmentally friendly because it can be used to replace non-reusable components of a mosquito repellent device.

### Brief description of the drawings

In the following, the invention will be described in detail. In the description, reference will be made to the appended drawings, in which
Fig. 1a shows, by way of example, a mosquito repellent component according to the invention in a diagonal view from above,
Fig. 1b shows the component of Fig. 1a in a side view,
Fig. 2a shows, by way of example, a preferred embodiment of a mosquito repellent component according to the invention in a so-called exploded view,
Fig. 2b shows, by way of example, another preferred embodiment of a mosquito repellent component according to the invention in a so-called exploded view,
Fig. 3 shows, by way of example, a cross-sectional view of a mosquito repellent device containing a mosquito repellent component according to the invention, and
Fig. 4 shows, by way of example, a mosquito repellent usage kit according to the invention.

### Detailed description of the invention

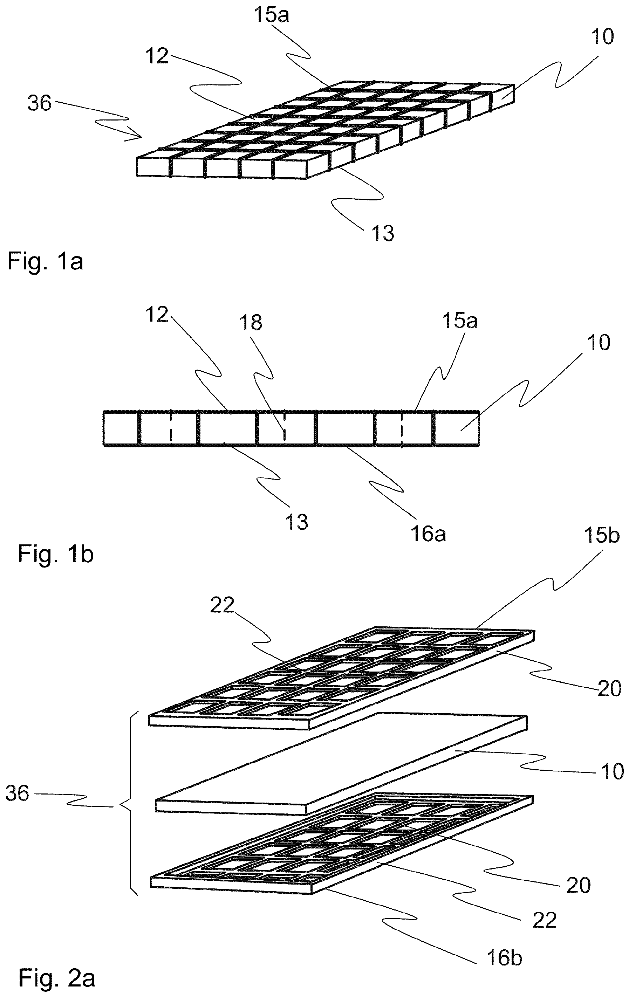

Figure 1a shows, by way of example, a mosquito repellent component 36 according to the invention in a diagonal view from above, and Fig. 1b shows the same component in a side view. In the following, both figures will be described at the same time.

The mosquito repellent component comprises a porous pad 10 made of a heatable felt-like material which can be impregnated with liquid mosquito repellent. The mosquito repellent may be any suitable repellent which has been found harmless to health in both liquid and vaporized state. Also, the repellent must withstand heating to the evaporating temperature, and it must not be ignited in liquid or vaporized state. Usable repellents include products containing prallethrin, D-allethrin or icaridine as the active ingredient.

The pad 10 shown in Figs. 1a and 1b is a felt-like piece cut in rectangular shape and having a first surface 12 and a second surface 13. The pad is made of an absorbent porous fabric, felt or fibreboard containing fibre material. The fibres in the pad may be natural fibres or synthetic fibres. In addition to the fibres, the pad may contain various fillers and/or adhesives. The combustion temperature or melting temperature of the pad is significantly higher than the evaporation temperature of the repellent absorbed in it. Preferably, the pad withstands the temperature of at least 200°C, preferably at least 300°C, most preferably at least 500°C, without breaking down and igniting. At least some of the fibres of the pad material may consist of incombustible material, such as mineral fibre or ceramic fibre. The size and thickness of the pad can be selected on the basis of the desired capacity to absorb repellent and/or according to the dimensions of the repellent device used. The absorption capacity of the pad will depend on, among other things, the size and number of pores and the viscosity of the repellent, whereby it is advisable to make sure that the pad material and the repellent absorbed in it act together in an appropriate way. The thickness of the pad may be, for example, 1 to 6 mm, preferably 2.5 mm, and the side dimensions of the pad may vary from a few centimetres to a few decimetres. Preferably, the pad has a width of approximately 34 mm and a height of 49, 96, or 144 mm.

The pad is enclosed in a supporting structure formed of a mesh made of metal wires, preferably steel wires, having a first wall 15a placed against the first surface of the pad, and a second wall 16a placed against the second surface of the pad. The first and second walls are connected to each other at the edges so that the metal wires of the walls extend around the edge surfaces of the pad; in other words, the supporting structure encircles the pad substantially on all sides. The thickness of the metal wires in the walls and the mesh size of the metal mesh can be selected, for example, according to the desired rigidity of the supporting structure. The diameter of the metal wires may be e.g. 0.5 to 2.0 mm, preferably 0.8 to 1.3 mm, and the mesh size of the metal mesh may be 5.0 to 20.0 mm, preferably 10.0 to 15.0 mm. Because the metal wires of the supporting structure are embedded in the surface of the porous pad, the supporting structure does not increase the outer dimensions of the component, but the outer dimensions of the component 36 are substantially equal to the outer dimensions of the pad.

The first and second walls are fastened to each other by fastening means 18 extending through the pad 10 and withstanding high temperatures and tensile stress. The fastening means may be metal wires and metal staples. By the fastening means, the walls are connected to each other so that they are pressed by a suitable force against the surfaces of the pad, whereby the pad and the supporting structure constitute a uniform sandwich structure staying together without external support. Thanks to the supporting structure, the component maintains its shape and stays together even if the adhesives in the pad lost their strength because of repeated heating of the component.

Figure 2a shows, by way of example, a preferred embodiment of a mosquito repellent component according to the invention in a so-called exploded view. In this embodiment, the pad 10 of the component is, with respect to its material as well as its chemical and technical properties, substantially equal to the embodiment shown in Figs. 1a and 1b, but the supporting structure surrounding the pad is different. In this embodiment, the first wall 15b and the second wall 16b of the supporting structure are separate elements which are connected to each other so that the pad is sandwiched between the walls. Both walls are substantially equal rigid pieces having a rectangular frame 20 and a mesh 22 that covers an opening limited by the frame. The walls are punched out of a metal sheet or are cast from a very heat resistant plastic or composite material so that the frame and the mesh constitute a uniform piece. The height of the frame in the thickness direction of the pad is slightly greater than the thickness of the mesh, whereby a suitable space for the pad is left between the first and second walls fastened to each other. The frames of the walls are equipped with compatible locking means, by which the walls can be clicked together (the locking means are not shown in the figures). The bending stiffness of the wires in the mesh is designed so that a slightly compressed pad sandwiched between the walls in the assembled component does not cause significant deformation of the meshes.

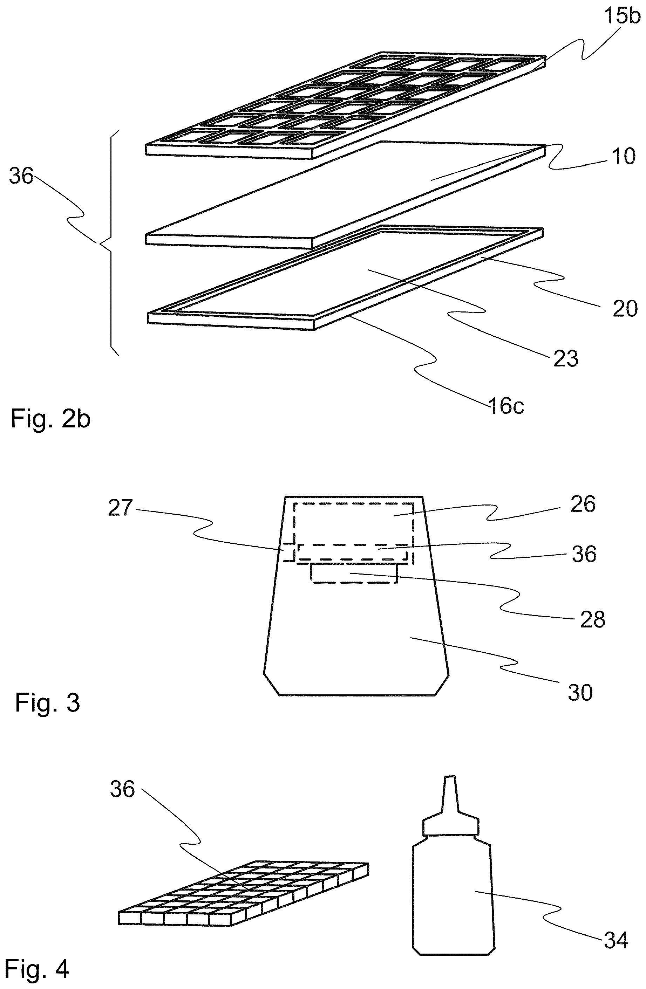

Figure 2b shows, by way of example, another preferred embodiment of a mosquito repellent component 36 according to the invention in an exploded view. In this embodiment, too, the pad 10 of the component is, with respect to its material as well as its chemical and technical properties, substantially identical to the embodiments shown in Figs. 1a, 1b and 2a, and the first wall 15b and the second wall 16c of the supporting structure surrounding the pad are separate elements which are connected to each other so that the pad is sandwiched between the walls. Both walls are substantially rigid rectangular pieces made of a very heat-resistant plastic or composite material by casting so that the frame and the mesh constitute a uniform casting. In this embodiment, the space limited by the frame 20 of the second wall is covered by a solid bottom 23 which is not permeable to liquid. The idea in this embodiment is that the frame and the bottom of the second wall constitute a liquid proof tray which prevents the liquid repellent absorbed in the pad from leaking through the second wall out of the component. Instead, any repellent possibly leaking from the pad will remain in the tray limited by the frame and the bottom, from which it is diffused by evaporation when the repellent device is used as will be described below. Naturally, the component must be installed in such a position in the repellent device that the bottom is placed in the lower part of the component.

In the embodiment shown in Fig. 2b, the bottom is an integral part of the second wall. However, the bottom may also be a separate, sheet-like piece equipped with a welted edge and placed between the mesh-like part 16a, 16b of the second wall and the second surface 13 of the pad 10 in the embodiments shown in Figs. 1a, 1b and 2a. Such a separate bottom may be made of, for example, thin metal foil.

Figure 3 shows, by way of example, a cross-sectional view of a mosquito repellent device 30 equipped with a mosquito repellent component according to the invention. The mosquito repellent device comprises a housing 32 with a space 26 for a mosquito repellent component 36. On a wall of the space, a heating device 28 is provided for heating the component, whereby the repellent absorbed in the component evaporates and diffuses into the surrounding air. The heating device may be electric and operated via mains, or preferably by batteries placed in the housing. The space 26 is closed by an openable cover, via which an empty component, from which the repellent has been exhausted, can be removed from the space and returned into the space after being filled with repellent. In an alternative, the wall of the housing may comprise a channel 27 which opens into the space 26 and via which the mosquito repellent component 36 can be inserted in the space and removed from the space.

Figure 4 shows, by way of example, a mosquito repellent usage kit according to the invention. The usage kit contains an embodiment of the above-presented mosquito repellent component 36 and a liquid package 34 containing liquid mosquito repellent compatible with the pad of the component. By providing the component and the liquid package containing the mosquito repellent in the same usage package, it is secured that a consumer purchasing the package can be sure of the functionality of the re-usable component.

Some advantageous embodiments of the mosquito repellent component and the mosquito repellent usage kit according to the invention have been described above. The invention is not limited to the solutions described above, but the inventive idea may be applied in different ways within the scope of the claims.

### List of reference numerals:

- 10: pad
- 12: first surface
- 13: second surface
- 15a, 15b: first wall
- 16a, 16b, 16c: second wall
- 18: fastening means
- 20: frame
- 22: mesh
- 23: bottom
- 26: space
- 27: channel
- 28: heating device
- 30: mosquito repellent device
- 34: liquid package
- 36: component

## Claims

1. A component (36) for a mosquito repellent device (30), comprising a heat resistant absorbent pad (10) for absorption and temporary storage of a mosquito repellent, and for diffusing it into the surrounding air, as well as a supporting structure protecting said pad (10) from mechanical stress, **characterized in that** said component (36) is re-usable; said pad (10) comprises a first surface (12) and a second surface (13); and said supporting structure has a first wall (15a, 15b) placed against the first surface (12) of the pad, and a second wall (16a, 16b, 16c) placed against the second surface (13) of the pad (10), wherein the first and second walls (15a, 15b, 16a, 16b, 16c) are permeable to liquid.

2. The component (36) according to claim 1, **characterized in that** said pad (10) is a flexible felt-like piece.

3. The component (36) according to claim 1 or 2, **characterized in that** the ignition or melting point of said pad (10) is higher than 300°C, preferably higher than 500°C.

4. The component (36) according to any of the claims 1 to 3, **characterized in that** said supporting structure and said pad (10) are made of different materials.

5. The component (36) according to any of the claims 1 to 4, **characterized in that** the ignition or melting point of said supporting point is at least as high as the ignition or melting point of said pad (10).

6. The component (36) according to any of the claims 1 to 5, **characterized in that** the first and/or the second wall (15a, 15b, 16a, 16b, 16c) comprises a mesh (22) having a planar shape, a plurality of thin rigid threads in substantially the same plane, and/or a perforated sheet.

7. The component (36) according to any of the claims 1 to 6, **characterized in that** said first and second walls (15a, 15b, 16a, 16b, 16c) are connected to each other by at least one fastening means (18) penetrating said pad.

8. The component (36) according to any of the claims 1 to 7, **characterized in that** said supporting structure encircles said pad (10) substantially entirely.

9. The component (36) according to any of the claims 1 to 8, **characterized in that** the rigidity and the bending strength of said supporting structure are higher than the rigidity and the bending strength of said pad (10).

10. The component (36) according to any of the claims 1 to 9, **characterized in that** said supporting structure is at least partly made of metal, silicone, a plastic resistant to a high temperature, or Teflon.

11. A usage kit for a mosquito repellent device (30), **characterized in that** it comprises a mosquito repellent component (36) according to any of the claims 1 to 10, and a liquid package (34) containing a liquid mosquito repellent.

12. The usage kit according to claim 11, **characterized in that** the pad (10) of said component (36) has a capacity to absorb mosquito repellent, and said liquid package (24) has a liquid volume, wherein the liquid volume is multiple, preferably at least ten-fold, most preferably at least 50-fold, with respect to said absorption capacity.

13. A mosquito repellent device (30) comprising a space (26) for housing a component (36) comprising volatile mosquito repellent and a heating device (28) for heating the component (36), **characterized in that** said component (36) is a component (36) according to any of the claims 1 to 10.
